# EUROPEAN PATENT APPLICATION

(11) **EP 1 543 814 A1**
(43) Date of publication of application: **22.06.2005**
(21) Application number: 04029794.7
(22) Date of filing: 16.12.2004
(51) Int. Cl.: A61K 7/06, A61K 7/075

(54) **Silicone-free hair care compositions providing long-lasting shine**

(30) Priority: 19.12.2003 US 741881
(71) Applicant: AVON PRODUCTS, INC., New York, NY 10020-1196 (US)
(72) Inventor: King, Ginger, Valley Cottage New York 10989 (US); Buckridge, Kenneth, Mahwah New Jersey 07430 (US)
(74) Representative: Weitzel, Wolfgang, Dr.-Ing.

(57) **Abstract**

The present invention provides hair care compositions and methods that impart shine and/or gloss to hair. The preferred compositions and methods of the present invention have one or more ethoxylated esters that are the reaction products formed by reaction of a saturated or unsaturated, preferably saturated, fatty acid having from about 8 to about 22 carbons with ethylene oxide and propylene oxide.

## Description

### BACKGROUND OF THE INVENTION

### 1. Field of the Invention

The present invention relates to hair care compositions. More particularly, the present invention relates to compositions and methods for imparting shine and/or gloss to hair. Still more particularly, the present invention relates to hair care compositions having one or more ethoxylated esters that provide increased shine, gloss and style retention to the hair, including scalp hair, eyebrows and eyelashes.

### 2. Description of the Related Art

Hair shine and/or gloss are attributes that are desired in many hair care products, preparations and compositions. Conventional hair shine/gloss products are made with silicones such as dimethicone, cyclomethicone, phenyltrimethicone and dimethicone copolyol. While these silicones offer very good shine due to their high refractive index, they also offer a heavy conditioning coating to the hair, which will interfere with the effect of styling products on the hair.

Traditional silicone-based shine products, depending on the molecular weight of the silicone used, either evaporate shortly after application, such as when cyclomethicone is used, or leave a heavy oily residue on the hair, such as when dimethicone is used. In these cases, the shine provided to the hair is either transient or may look and feel greasy.

Silicones are known to be used as plasticizers in hair stylers to prevent brittleness of the film, yet any modification of a styler with silicones tends to weaken the holding power of the styler. Silicones are heavy, and their presence in hair care compositions weigh the hair down. Also, the use of volatile silicones results in hair care compositions that provide a fleeting shine, and a tendency to soften hair after application, resulting in a style that does not last long. Moreover, silicone-based hair compositions tend to be costly.

Until now, ethoxylated esters have been known in the art as emollients and hair conditioning agents, not as shine agents or glossers. The present invention provides hair care compositions having one or more ethoxylated esters as a shine agent to replace silicones, yet provide long-lasting shine and moisturization, without the greasy feel or residue commonly associated with silicone products, and without weakening the hold power as dramatically as traditional silicone products. Since the present invention is aqueous-based, it also provides a greater cost benefit over the more costly silicone.

### SUMMARY OF THE INVENTION

It is an object of the present invention to provide a composition that has one or more ethoxylated esters as herein recited in an amount effective to increase shine and/or gloss to hair, including scalp hair, eyebrows, and eyelashes.

It is another object of the present invention to provide a composition that is silicone-free or virtually silicone-free.

It is yet another object of the present invention to provide a composition that has an aqueous base.

It is still another object of the present invention to provide a composition that provides style retention to the hair.

It is still yet another object of the present invention to provide a composition that is clear in color.

It is a further object of the present invention to provide a composition that is a leave-on hair gel.

It is still a further object of the present invention to provide a method of imparting shine and/or gloss to the hair, including scalp hair, eyebrows, and eyelashes, comprising topically applying to hair an aqueous-based, silicone-free composition having one or more of the ethoxylated esters of the present invention in an amount effective to increase shine and/or gloss.

These and other objects and advantages of the present invention are achieved by a composition having one or more ethoxylated esters of the present invention. The ethoxylated esters for use in the compositions of this invention are the reaction products formed by reaction of a saturated or unsaturated, preferably saturated, fatty acid having from about 8 to about 22 carbons with ethylene oxide and propylene oxide. Accordingly, the esters of the present invention have both ethoxylated and propoxylated groups. The one or more ethoxylated esters are present in an amount effective to provide benefits including shine, gloss, and style retention in hair, including scalp hair, eyebrows, and eyelashes. The total amount of ethoxylated esters is about 0.1 wt% to about 40 wt% based on the total weight of the composition. The composition is preferably clear, aqueous-based and silicone-free or virtually silicone-free. The preferred ethoxylated ester is PEG/PPG-8/3 Laurate.

The present invention also provides a method of increasing shine, gloss, and style retention in hair. The method includes topically applying to hair an effective amount of ethoxylated ester.

Other and further objects, advantages and features of the present invention will be understood by reference to the following.

### BRIEF DESCRIPTION OF THE DRAWINGS

This patent or application contains at least one drawing executed in color. Copies of this patent or patent application publication with color drawing(s) will be provided by the Office upon request and payment of the necessary fee.
Fig. 1 is a color photograph comparing shine levels in hair tresses treated with four different hair shine compositions versus a control at 0 hours;
Fig. 2 is a color photograph comparing shine levels in hair tresses treated with four different hair shine compositions versus a control at 2 hours;
Fig. 3 is a color photograph comparing shine levels in hair tresses treated with four different hair shine compositions versus a control at 18 hours; and
Fig. 4 shows style retention results measured by percent delta increase over a four hour period demonstrated for two different compositions relative to a control described in Example 2 below.

### DESCRIPTION OF THE INVENTION

The present invention relates to compositions that provide shine and/or gloss to hair, and methods for providing shine and/or gloss to hair. The compositions and methods of the present invention provide for long-lasting shine without the presence of residue, while at the same time outperforming conventional silicone-based products on hold and style retention.

The present compositions have one or more ethoxylated esters that are the reaction products formed by reaction of a saturated or unsaturated, preferably saturated, fatty acid having from about 8 to about 22 carbons with ethylene oxide and propylene oxide. It is believed that such esters exhibit both water solubility and oil solubility. The ethoxylated ester is used as a shine agent or glosser, and replaces the silicones previously used in order to produce shine in hair care compositions. The ethoxylated esters are present in an amount effective to provide and/or enhance shine and/or gloss to scalp hair, as well as eyelash hair and eyebrow hair.

Ethoxylated esters are known in the art as emollients and hair conditioning agents, not as shine agents or glossers. In fact, esters, and especially ethoxylated esters, are not known to have silicone type of shine. Typically, ethoxylated esters have been used in the art in make-up foundations to aid in pigment dispersion. Surprisingly, the present compositions having ethoxylated esters not only gave shine, but the shine lasted longer than conventional silicone-based products. It was also surprisingly found that the use of ethoxylated esters not only achieved long-lasting shine, but also did not result in the greasiness associated with conventional silicone products. It is also believed that the ethoxylated esters will not plasticize styling products, thus desired hair styles will last longer.

The ethoxylated ester is formed by reaction of a saturated or unsaturated, preferably saturated, fatty acid having from about 8 to about 22 carbons, preferably 14 to 18 carbons, with ethylene oxide and propylene oxide. Thus, the ethoxylated esters have both an ethoxylated moiety and a propoxylated moiety. Preferably, the esters have a degree of ethoxylation of at least 5 mols of ethylene oxide and a degree of propoxylation of at least one mol of propylene oxide. The more preferred esters of this invention have a degree of ethoxylation that is greater than the degree of propoxylation. A most preferred ethoxyalted ester in accordance with this invention is PEG/PPG-8/3 Laurate.

Most preferably, the ethoxylated ester used is PEG/PPG-8/3 Laurate (Polyethylene Glycol 400 Propoxylated Monolaurate). PEG/PPG-8/3 Laurate is oil and water soluble. It has the following chemical structure: 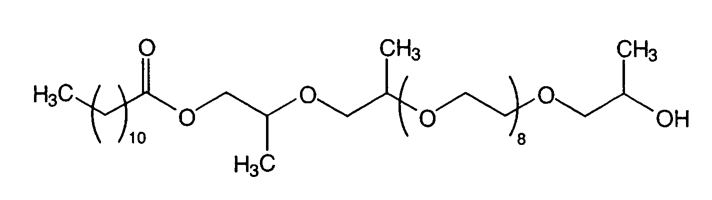 PEG/PPG-8/3 Laurate can be added directly into a vehicle for a water-based shine gel or other hair care composition, without any dilution that is normally needed for silicones. This vehicle is preferably aqueous based. Thus, the use of ethoxylated ester enables the formulators to more easily formulate the present compositions than a conventional silicone product, and at a fraction of the cost of a traditional silicone product. Further, the present compositions are preferably clear and virtually silicone-free. "Virtually silicone-free" means that the silicone content of the composition is less than about 0.001 wt% by weight of the total composition, preferably less than 0.0001 wt% by weight of the total composition.

Solubility is the ability of one substance to blend uniformly with another, e.g. solid in liquid or liquid in liquid. Clarity is determined visually. A turbidity meter may be used to measure clarity if the system is not absolutely clear. The solubility of PEG/PPG-8/3 Laurate is up to fifty percent (50%) in water. In water, it is clear and homogeneous at concentrations up to its solubility limit. The solubility of PEG/PPG-8/3 Laurate is up to ten percent (10%) in sunflower oil and in castor oil. In sunflower and castor oil, it is also clear and homogenous up to its solubility limit. In soybean oil, it is clear at five percent (5%) and slightly hazy at ten percent (10%).

Preferably, the one or more ethoxylated esters are used in conjunction with a compatible vehicle to form the present compositions. A compatible vehicle is a vehicle that does not unduly interfere with, negatively affect, or mask shine over time. Preferably, the vehicle used is a clear and cosmetically acceptable vehicle. Examples of vehicles that are suitable for the present compositions include, but are not limited to, gel, spray, liquid serum, pomade, stick, solution, and microemulsion. Gel, solution, liquid serum, spray and microemulsion are preferred. The one or more ethoxylated esters can also be placed in mineral oil. As such, the ethoxylated ester can be administered as part of a leave-on hair gel, spray, liquid serum, pomade, stick, solution, microemulsion, or other analogous hair care product.

The compositions of the present invention will have the one or more ethoxylated esters in an amount that totals about 0.1 percentage by weight or weight percent (wt%) to about 40 wt% based on the total weight of the composition. The ethoxylated esters are preferably present in an amount about 1 wt% to about 10 wt%, and more preferably present in an amount about 1 wt% to about 5 wt%, based on the total weight of the composition. Most preferably, the ethoxylated esters are present in an amount about 2 wt% based on the total weight of the composition.

Preferably, the hair care compositions of the present invention have PEG/PPG-8/3 Laurate. The PEG/PPG-8/3 Laurate exhibits an index of refraction of about 1.456. Refractive index impacts the overall clarity of the present composition. It is preferred to have an aqueous-based composition with a clear color for aesthetic appeal. The hair care compositions of the present invention have a Shine Index of at least 5 hours, preferably about 8 or more hours, and most preferably up to 18 hours or more, thereby providing for extended life shine. 'Shine Index" as used herein is the length of time shine persists on treated hair, as evaluated in the test protocol described in Example 1.

The present compositions may have additional ingredients suitable for hair care products. Examples of such additional ingredients include, but are not limited to, one or more buffers, chelating and sequestering agents, colorants, emollients, film formers, fragrances, humectants, hair conditioning agents, pH adjusting agents, propellants, preservatives, surfactants, viscosity modifiers, and viscosity control agents.

The compositions of the present invention may also include other active agents. Such other active agents may include, but are not limited to: one or more insect repellants; hair colorants; sunscreens; UV light absorbers; antidandruff agents; antimicrobial and antifungal agents; biological and plant extracts; and self-tanning agents, such as for example dihydroxyacetone.

The present compositions may be formulated in any convenient form suitable for topical application to the hair, eyelashes, and eyebrows.

### EXAMPLES

The effect of an aqueous-based composition of the present invention having PEG/PPG-8/3 Laurate (ethoxylated ester) on hair shine and/or gloss, as well as on hair style retention, is demonstrated below in comparison to a control and various comparative silicone-based compositions.

### Example 1-Hair Shine Evaluation Test

A hair shine evaluation test was performed to accurately rate the level of shine that a product gives to hair. This test allows the evaluator to distinguish between a matte product, a medium glossy product, and a very glossy product.

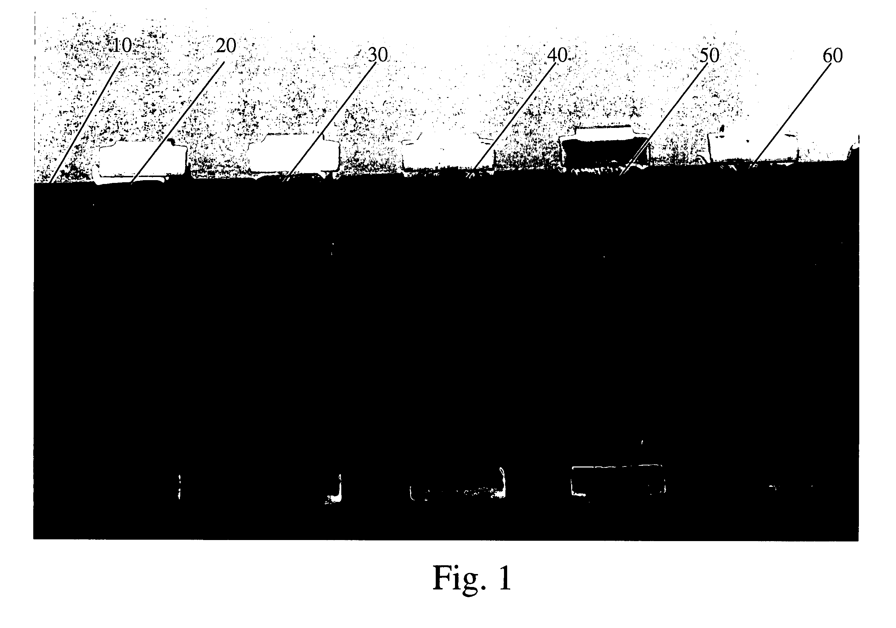

As illustrated in Figs. 1 to 3, five dark brown hair tresses 20, 30, 40, 50, and 60 were tested. Four of the tresses were treated with four different hair shine compositions and all were observed for eighteen (18) hours. Hair tress 20 was the control, and included only dark brown hair. No shine composition was applied to hair tress 20. The remaining tresses, 30, 40, 50 and 60, were treated and all were fastened to a revolving tress roller or shine bar 10.

The dark brown hair of hair tress 30 was sprayed with Comparative Composition A that comprised a shine spray having the following ingredients: SD alcohol 40-B; cyclomethicone; phenyl trimethicone; fragrance; panthenyl ethyl ether; phytantriol; wheat amino acids; hydrolyzed wheat protein; wheat oligosaccharides; and potassium sorbate. Comparative Composition A is a cyclomethicone-based formula. 1.0g of the shine spray Composition A was applied evenly across the hair on top, middle and bottom section, combed through, and hair tress 30 was mounted to shine bar 10.

The dark brown hair of hair tress 40 was applied with Comparative Composition B that comprised a shine polisher having the following ingredients: water; cyclomethicone; alcohol denat.; dimethicone; PVP/VA copolymer; methacryloyl ethyl betaine/acrylates; copolymer; polyacrylamide; C13-14 isoparaffin; laureth-7; amodimethicone; dimethicone copolyol; moanalua (citrus grandis) essence; pummelo (citrus grandis) essence; tangerine (citrus reticulata) essence; tahiti lime (citrus latifolia) essence; disodium EDTA; DMDM hydantoin; and fragrance. Comparative Composition B is a silicone emulsion composed of cyclomethicone, dimethicone, amodimethicone and dimethicone copolyol. 1.0g of the shine polisher Composition B was applied evenly across the hair on top, middle and bottom section, combed through, and hair tress 40 was mounted to shine bar 10.

The dark brown hair of hair tress 50 was applied with Composition C that comprised a shine glaze composition of the present invention having the following ingredients: water; propylene glycol; butylene glycol; glycerin; PEG/PPG-8/3 Laurate; PPG-5-Ceteth-20; carbomer; aminomethyl propanol; fragrance; tetrasodium EDTA; creatine; laminaria digitata extract; and pelvetia canaliculata extract. Composition C is a silicone-free shine formula of the present invention. 1.0g of the shine glaze Composition C was applied evenly across the hair on top, middle and bottom section, combed through, and hair tress 50 was mounted to shine bar 10.

The dark brown hair of hair tress 60 was applied with Comparative Composition D that comprised a shine serum composition having the following ingredients: propylene glycol; cyclomethicone; water; hexylene glycol; phenyl trimethicone; dimethicone; polyacrylamide; laureth-7; C13-14 isoparaffin; octyl methoxycinnamate; fragrance; phenoxyethanol; and methyldibromo glutaronitrile. Comparative Composition D is a formula that contains cyclomethicone, phenyltrimethicone and dimethicone. 1.0g of the shine serum Composition D was applied evenly across the hair on top, middle and bottom section, combed through, and hair tress 60 was mounted to shine bar 10.

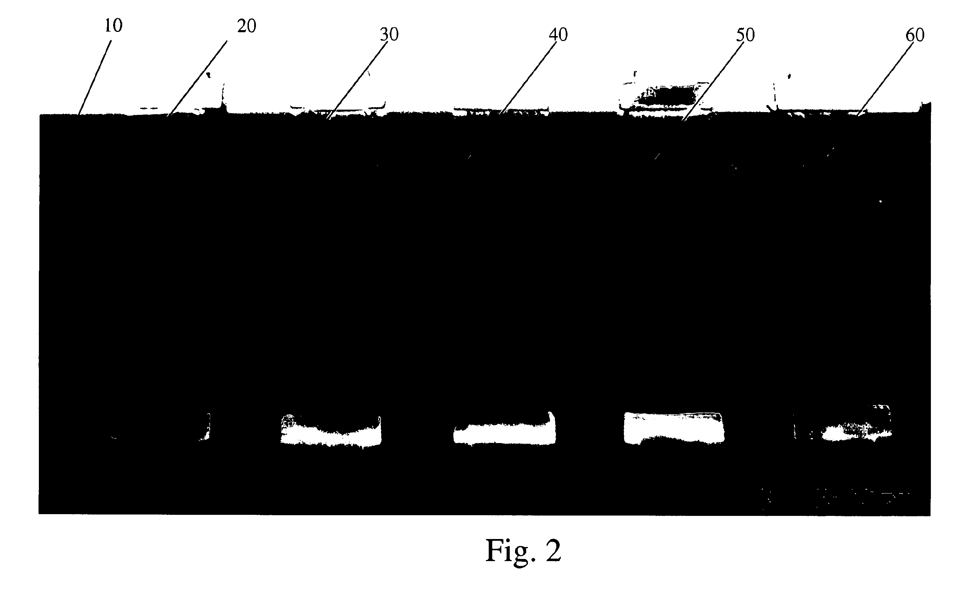

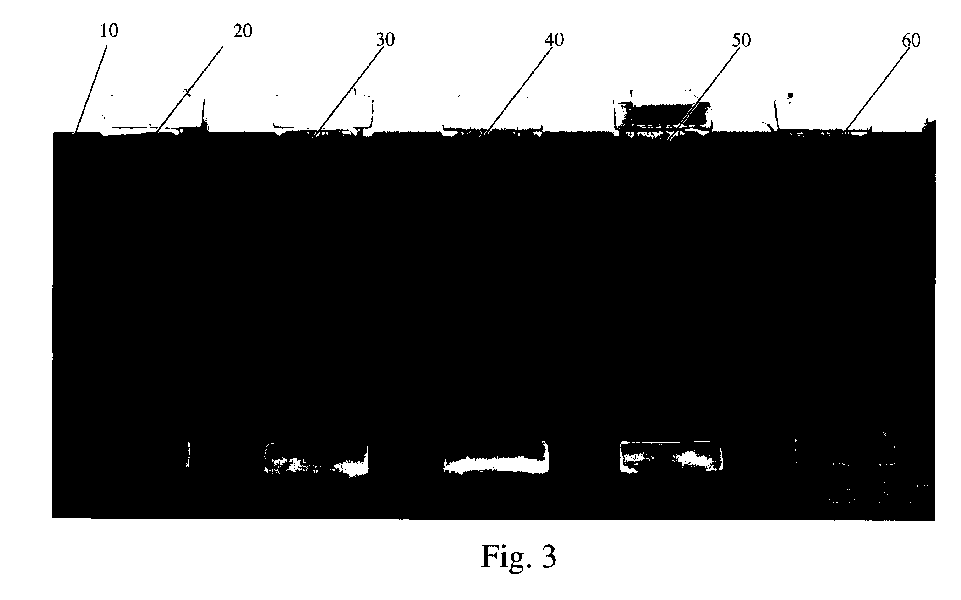

Fig. 1 illustrates the initial shine results for tresses 20, 30, 40, 50, and 60, immediately after application of Comparative Compositions A, B, C, and D to tresses 30, 40, 50, and 60, respectively, under controlled shine box lighting (setting: UV and sunlight) at zero hours. Gloss of each tress is evaluated at the top middle of the tress. There are typically one to two bands of gloss in this area. The more gloss the hair has, the more defined the two bands will be. Furthermore, shine is judged by the narrowest "shine band" reflected. As shown in Fig. 1, there is no major difference between all tested products initially. Fig. 2 illustrates that after two hours, tress 40 with Comparative Composition B started to look dull, while tress 50 with Composition C of the present invention still remained shiny. Finally, as illustrated in Fig. 3, after eighteen hours, tress 50 with Composition C of the present invention appeared to remain shiny and to outlast all other compositions at the end of the eighteen-hour period. Furthermore, when the hair tresses were removed from shine bar 10, there was oily residue found on the bar section that mounted tress 60.

### Example 2-Delta Angle Analysis

Delta angle analysis was used to predict hair style retention after four (4) hours in high humidity condition (85rh, 37C). Delta angle analysis can precisely measure the movement of the curled swatch in degrees. A one (1) inch difference on the curl retention board is equal to ten (10) degrees difference on the delta angle chart. As a general rule, a styling product will have good style retention after four (4) hours if the percent delta angle increase is less than 100%. A control is always used for this study. If the percent delta angle difference lies between 40% to 100%, the styling product has great style retention ability. If the percent delta angle difference lies between 100% and 200%, it is considered good. Any increase over 200% is most likely to perform poorly under high humidity condition.

The materials typically used for this analysis are as follows: low density double bleached hair from International Hair Importers; rollers (2 inches wide in diameter); roller clips (3 per roller); comb; styrofoam backed poster board; push pins; medium size binder clips; tape; protractor; ruler; and circle templates.

The procedure typically followed for delta angle analysis is set forth below. A hair swatch (1 inch wide, 6 inches long) is wet under running tap water (35C to 40C) for fifteen (15) seconds. The hair swatch is then combed with the wide tooth comb on the hair eight (8) times alternating front and back to get rid of tangles. The water is squeezed out with the second and third fingers three (3) times. The styling product is distributed evenly. (0.5 gram is used for gel, 1.0 gram for mousse). The hair is combed through four (4) times alternating front and back. The hair is rolled tight on 2 inch diameter rollers and one is clipped on the top, one on the middle and one at the end of the tress. The rolls wrapped with hair swatch are placed in 120F oven for two (2) hours for drying. The rolls are next taken out of the oven and left out to equilibrate to room temperature for at least thirty (30) minutes. Using the circle template, the templates are taped onto the poster board, ensuring that the tops are level. At the 0 degree line, a pushpin is inserted all the way through. The 0 axis should be hanging vertically, with the 90 degree axis running horizontally. The top of the binder clip is placed sideways over the pushpin with the pushpin going through the triangular opening of the binder clip. It is secured in place with tape. These steps are repeated for all samples including a control. After the swatches are equilibrated to RT, they are removed carefully from the rollers and hanged. The swatches are all hung at the same level, using the pushpin as a guide. The swatches hanged so that the curl opens facing the template and so that one can view the side of the curl. The board is placed in high humidity chamber (85RH, 37C). A "dot" is placed with a marker where the end of the curl is on the template. This is repeated for each tress. The curls are checked again at 15, 30, 60, 90, 120, 180 and 240 minutes. A dot is marked at the end of the curl. The swatches are taken off from the board and all graph sheets are collected. A line is drawn from the center of the graph through each dot. A protractor is used to measure the angle of the line as compared to the 0 degree line. The degree is recorded on paper, the data is input into EXCEL computer program and the average, standard deviation, delta angle difference and the percent delta angle increase (the smaller increase the longer the "hold" time) are computed. The less percentage changes in delta angle, the better hold retention a product can provide.

The control composition E used for this particular analysis comprised a hair spray having the following ingredients: SD alcohol 40; water; dimethyl ether; isobutane; acrylate dimethicone copolymer; panthenol; isododecane; potassium hydroxide; diisobutyl adipate; sodium benzoate; and fragrance. The first test (Test 1) applied control composition E in addition to a silicone-based shine serum composition having the following ingredients: propylene glycol; cyclomethicone; water; hexylene gylcol; phenyl trimethicone; dimethicone; polyacrylamide; laureth-7; C13-14 isoparaffin; octyl methoxycinnamate; fragrance; phenoxyethanol; and methyldibromo glutaronitrile. The second test (Test 2) applied control composition E in addition to a silicone-free shine glaze composition of the present invention having the following ingredients: water; propylene glycol; butylene glycol; glycerin; PEG/PPG-8/3 Laurate; PPG-5-Ceteth-20; carbomer; aminomethyl propanol; fragrance; tetrasodium EDTA; creatine; laminaria digitata extract; and pelvetia canaliculata extract.

For the control, control composition E was sprayed evenly on the top, center, bottom, front and back on three (3) bleached blond hair swatches. The hair was rolled in a roller and placed in an oven for one (1) hour to dry. The delta angle method outlined above was then performed.

For Test 1, one (1) gram of the Test 1 shine composition was applied evenly on three (3) bleached blond hair swatches. The hairspray was then applied as outlined above with respect to the control. The hair was rolled in a roller and placed in an oven for one (1) hour to dry. The delta angle method outlined above was then performed.

For Test 2, one (1) gram of the Test 2 shine composition was applied evenly on three (3) bleached blond hair swatches. The hairspray was then applied as outlined above with respect to the control. The hair was rolled in a roller and placed in an oven for one (1) hour to dry. The delta angle method outlined above was then performed.

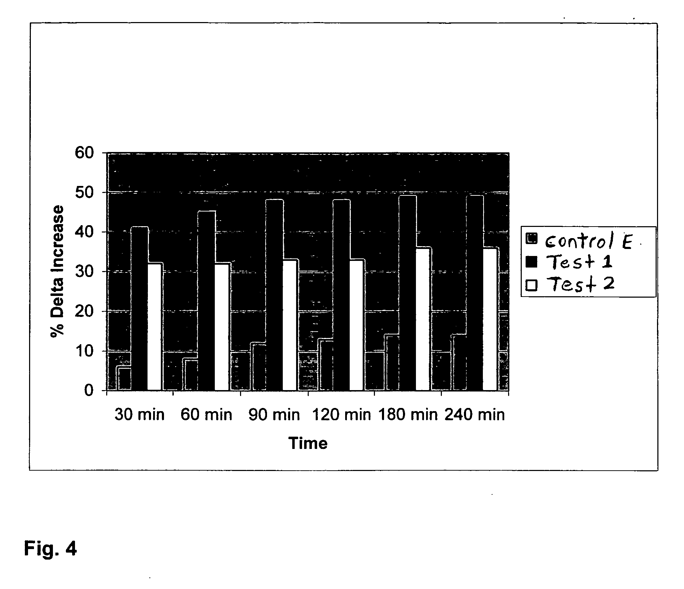

Fig. 4 illustrates that the shine composition of the present invention having an ethoxylated ester provides style retention more effectively than traditional silicone-based shine glaze consistently over a four-hour test period. The lower bars on the graph indicate better shape retention.

Delta Angle Analysis shows that the present invention outperforms conventional silicone-based products on hold and style retention.

### Example 3

The following is an example of a silicone-free aqueous-based shine glaze hair care composition having an ethoxylated ester according to the present invention.

| Ingredients | WT % |
|---|---|
| Glycols | 10 - 40 |
| Glycerin | 2 - 10 |
| Ethoxylated ester (e.g., PEG/PPG-8/3 Laurate) | 1- |
| PPG-5-Ceteth-20 | 0.5 - 2.5 |
| Carbopol 940 | 0.25 - 1.5 |
| Aminomethyl Propanol-95% | 0.25 - 1.5 |
| Fragrance | 0.2 - 0.6 |
| Water | QS |

It should be understood that the foregoing description and examples are only illustrative of the present invention, and are not offered as limitations. Various alternatives and modifications can be devised by those skilled in the art without departing from the present invention. Accordingly, the present invention is intended to embrace all such alternatives, modifications and variations that fall within the scope of the appended claims.

## Claims

1. A composition for the hair comprising:
one or more ethoxylated esters in an amount effective to increase shine and/or gloss, the ethoxylated esters being the reaction products formed by reaction of a saturated or unsaturated fatty acid having from 8 to 22 carbons with ethylene oxide and propylene oxide; and
a clear and cosmetically acceptable vehicle.

2. The composition of claim 1, wherein the composition has an aqueous base.

3. The composition of claim 1, wherein the composition is silicone-free.

4. The composition of any of claims 1-3, wherein the composition is clear in color.

5. The composition of any of claims 1-3, wherein the fatty acid is saturated and has 14 to 18 carbons.

6. The composition of any of claims 1-3, wherein said one or more esters have a degree of ethoxylation and a degree of propoxylation, wherein said degree of ethoxylation is greater than said degree of propoxylation.

7. The composition of any of claims 1-3, wherein said one or more esters have a degree of ethoxylation of at least 5 mols of ethylene oxide and a degree of propoxylation of at least one mol of propylene oxide.

8. The composition of any of claims 1-3, wherein said one or more ethoxylated esters is PEG/PPG-8/3 Laurate.

9. The composition of any of claims 1-3, wherein said one or more ethoxylated esters are present in an amount 0.1 wt% to 40 wt% based on the total weight of the composition.

10. The composition of any of claims 1-3, wherein said one or more ethoxylated esters are present in an amount 1 wt% to 10 wt% based on the total weight of the composition.

11. A method of imparting shine and/or gloss to hair, comprising topically applying to hair a composition having one or more ethoxylated esters in an amount effective to increase shine and/or gloss to the hair, the ethoxylated esters being the reaction products formed by reaction of a saturated or unsaturated fatty acid having from 8 to 22 carbons with ethylene oxide and propylene oxide.

12. The method of claim 11, wherein said composition is an aqueous-based composition.

13. The method of claim 11, wherein said composition is silicone-free.

14. The method of any of claims 11-13, wherein the fatty acid is saturated and has 14 to 18 carbons.

15. The method of any of claims 11-13, wherein said one or more esters have a degree of ethoxylation and a degree of propoxylation, wherein said degree of ethoxylation is greater than said degree of propoxylation.

16. The composition of any of claims 11-13, wherein said one or more esters have a degree of ethoxylation of at least 5 mols of ethylene oxide and a degree of propoxylation of at least one mol of propylene oxide.

17. The method of any of claims 11-13, wherein said one or more ethoxylated esters is PEG/PPG-8/3 Laurate.
